(19) Europäisches Patentamt
European Patent Office
Office européen des brevets

(11) Veröffentlichungsnummer: **0 012 982**
**B1**

(12) # EUROPÄISCHE PATENTSCHRIFT

(45) Veröffentlichungstag der Patentschrift: **18.05.83**

(51) Int. Cl.³: **C 07 C 143/833,**
C 07 C 127/00

(21) Anmeldenummer: **79105241.8**

(22) Anmeldetag: **18.12.79**

(54) **N-Nitroaryl-N'-halogensulfonylharnstoffe, Verfahren zu ihrer Herstellung und ihre Verwendung zur Herstellung von Nitroarylharnstoffen.**

(30) Priorität: **23.12.78 DE 2855884**

(43) Veröffentlichungstag der Anmeldung:
**09.07.80 Patentblatt 80/14**

(45) Bekanntmachung des Hinweises auf die Patenterteilung:
**18.05.83 Patentblatt 83/20**

(84) Benannte Vertragsstaaten:
**BE CH DE FR GB IT**

(56) Entgegenhaltungen:
**FR - A - 1 237 963**
**US - A - 3 996 208**

(73) Patentinhaber: **HOECHST AKTIENGESELLSCHAFT**
**Postfach 80 03 20**
**D-6230 Frankfurt/Main 80 (DE)**

(72) Erfinder: **Arndt, Otto, Dr.**
**Frankfurter Strasse 38**
**D-6238 Hofheim am Taunus (DE)**
Erfinder: **Tronich, Wolfgang, Dr.**
**Martin-Wohmann-Strasse 27**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 012 982 B1

# N-Nitroaryl-N'-halogensulfonylharnstoffe, Verfahren zu ihrer Herstellung und ihre Verwendung zur Herstellung von Nitroarylharnstoffen

Gegenstand der Erfindung sind N-Nitroaryl-N'-halogensulfonylharnstoffe der allgemeinen Formel I

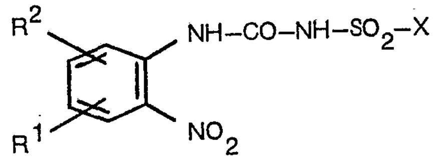

I

in der X Fluor oder Chlor, $R^1$ Wasserstoff oder Nitro und $R^2$ Wasserstoff, Fluor, Chlor, Brom, niederes Alkyl, niederes Alkoxy, Trifluormethyl, Cyan, Phenyl oder ein ankondensierter Benzolring ist.

In bevorzugten erfindungsgemäßen Verbindungen steht X für Chlor und $R^2$ für Wasserstoff, Chlor, Methyl, Methoxy, Cyan oder einen ankondensierten Benzolring.

Gegenstand der Erfindung ist weiterhin ein Verfahren zur Herstellung der Verbindungen der Formel I, das dadurch gekennzeichnet ist, daß man ein Amin der allgemeinen Formel II

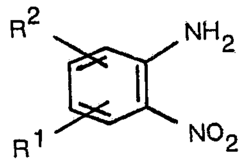

II

in der $R^1$ und $R^2$ die vorstehend angegebenen Bedeutungen haben, mit Fluor- oder vorzugsweise Chlorsulfonylisocyanat, bevorzugt in einem inerten organischen Lösemittel, umsetzt.

Gegenstand der Erfindung ist weiterhin die Verwendung der Verbindungen der Formel I zur Herstellung der entsprechenden Nitroarylharnstoffe der Formel III,

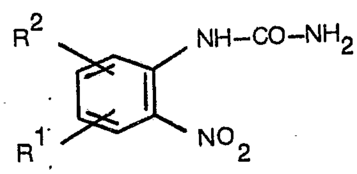

III

in der $R^1$ und $R^2$ die vorstehend genannten Bedeutungen haben, durch Hydrolyse.

In der FR - A - 1.237.963 ist Anilin als Ausgangsmaterial für die Umsetzung mit Chlorsulfonylisocyanat genannt. Gegenüber dem dabei erhaltenen Reaktionsprodukt unterscheiden sich die erfindungsgemäßen Verbindungen durch die ortho-ständige Nitrogruppe. Nitrosubstituenten sind in dieser Druckschrift nicht genannt.

In der US - A - 3.996.208 ist p-Nitrophenyl als aromatischer Rest eines Amins genannt. Aus p-Nitroanilin würde in Analogie zum Beispiel 40 ein Stellungsisomeres zum Produkt des erfindungsgemäßen Beispiels 10 erhalten.

Gegen eine Übertragung dieser bekannten Reaktion auf die entsprechenden ortho-Nitroverbindungen bestand jedoch für den Fachmann am Prioritätstag der Erfindung ein erhebliches Vorurteil:

Aus J. pr. Ch. [2] *59*, 266 (1899), Seite 280, war es bekannt, daß von den Nitroanilinen "nur das meta-Isomere glatt mit Kaliumcyanat Resultate" ergab, während das ortho-Isomere überhaupt nicht reagierte.

Es wird ausdrücklich erwähnt, daß diese Beobachtung im Einklang mit mehreren anderen steht, die über die Wirkungsweise von o-Nitroanilin gemacht worden sind.

In die gleiche Richtung geht J. Ind. Chem. Soc. 7 (1930) 793, wo auf Seite 794, Zeile 2 ebenfalls die Ausnahmestellung von aromatischen ortho-Nitroaminen hervorgehoben wird. Im experimentellen Teil wird unter den Ziffern 4 und 5 berichtet, daß m- und p-Nitroanilin die entsprechenden Dinitrodiphenylharnstoffe mit Harnstoff liefern.

Im folgenden werden weitere bevorzugte Ausgestaltungen der Erfindung näher erläutert:

Die Umsetzung des Amins der Formel II mit dem Halogensulfonylisocyanat kann, insbesondere bei flüssigen Aminen, ohne Löse- oder Verdünnungsmittel durchgeführt werden, bevorzugt wird jedoch die Umsetzung in einem inerten Löse- oder Verdünnungsmittel durchgeführt, wobei das Amin der Formel II in diesem Reaktionsmedium suspendiert oder gelöst sein kann.

Als Löse- oder Verdünnungsmittel kommen alle gegen die Reaktionsteilnehmer inerten Flüssigkeiten in Betracht, wie aliphatische und aromatische unsubstituierte oder substituierte Kohlenwasserstoffe oder Heterocyclen, wie Tetramethylensulfon oder cyclische Äther. Bevorzugt werden aromatische Kohlenwasserstoffe wie Xylole, Chlorbenzol, Dichlorbenzole, insbesondere jedoch Toluol. Werden wasserhaltige Löse- oder Verdünnungsmittel oder Amine eingesetzt, so muß vor der Reaktion mit dem Halogensulfonylisocyanat selbstverständlich eine Entwässerung, beispielsweise durch azeotrope Destillation, durchgeführt werden.

Das Halogensulfonylisocyanat wird vorzugsweise in einem geringen Überschuß eingesetzt.

Die Reaktionstemperatur liegt vorzugsweise zwischen Raumtemperatur und dem Siedepunkt des verwendeten Löse- bzw. Verdünnungsmittels, insbesondere zwischen 40 und 100°C. Die Reaktionsdauer beträgt in Abhängigkeit von der Reaktionstemperatur im allgemeinen zwischen 2 und 10 Stunden.

Das Verfahrensprodukt der Formel I kann aus dem Reaktionsmedium, vorzugsweise bei relativ tiefen Temperaturen, isoliert werden und mit dem Reaktionsmedium oder einem anderen inerten Lösemittel gewaschen werden. Eine Charakterisierung kann beispielsweise mit Hilfe der Kernresonanzspektren erfolgen.

Für die erfindungsgemäße Verwendung des Verfahrensproduktes ist eine Isolierung jedoch nicht erforderlich und bringt — da das Verfahrensprodukt feuchtigkeitsempfindlich ist — keine Vorteile. Vorzugsweise wird deshalb das Verfahrensprodukt ohne Isolierung zu den entsprechenden Nitroarylharnstoffen hydrolysiert. Durch diese Verseifung zum Arylharnstoff kann auch eine Charakterisierung der Verfahrensprodukte erfolgen.

Bevorzugt erfolgt die Verseifung durch Zugabe von Wasser zum Reaktionsgemisch, was im allgemeinen bei Temperaturen unter 25°C erfolgt. Die eigentliche Verseifung wird bei etwa 10 bis etwa 100°C, vorzugsweise bei 50 bis 80°C durchgeführt, wobei jedoch auch andere Verseifungstemperaturen möglich sind.

Die Dauer der Verseifung ist temperaturabhängig und beträgt im allgemeinen 3 bis 5 Stunden.

Die Hydrolyse kann in einem pH-Bereich zwischen etwa 1 und 9 vorgenommen werden, wobei der saure Bereich bevorzugt ist.

Der Nitroarylharnstoff kann unmittelbar aus dem Reaktionsgemisch oder nach vorhergehender Neutralisation der gebildeten Halogenwasserstoffsäure und Schwefelsäure isoliert, beispielsweise abfiltriert werden. Es ist jedoch auch möglich, zunächst aus der neutralisierten Suspension das organische Lösemittel, beispielsweise durch Destillation mit Wasserdampf, zu entfernen. Das organische Lösemittel kann dann entweder in regenerierter Form oder als Mutterlauge mehrmals für das erfindungsgemäße Verfahren eingesetzt werden.

Die erfindungsgemäßen Verbindungen sind aufgrund der reaktionsfähigen Halogensulfonylgruppe zu einer Vielzahl von weiteren Reaktionen befähigt. Von Vorteil ist, daß diese Folgeprodukte als weitere funktionelle Gruppe eine oder zwei Nitrogruppen enthalten, die diese Folgeprodukte direkt oder nach Abwandlung der Nitrogruppen in Aminogruppen vielseitig verwendbar macht.

Bei der erfindungsgemäßen Verwendung werden bekannte Nitroarylharnstoffe erhalten. Diese Verbindungen können durch Reduktion, insbesondere katalytische Hydrierung, der Nitrogruppen in die Mono- und Diaminophenyl- bzw. -naphthylharnstoffe übergeführt werden und daraus durch Ringschluß unter Ammoniakabspaltung die entsprechenden Benzimidazolone bzw. Naphthimidazolone hergestellt werden. Wenn man von den Dinitroarylharnstoffen ausgeht, werden so in einfacher Weise die Aminobenzimidazolone bzw. Amino-naphthimidazolone erhalten, die als Diazokomponenten und nach Umsetzung, beispielsweise mit Diketen, zur N-Acetoacetyl-Verbindung als Kupplungskomponenten für Azopigmente verwendbar sind.

In den folgenden Beispielen beziehen sich Teile und Prozentangaben auf das Gewicht, sofern nichts anderes angegeben ist.

### Beispiel 1

311 Teile 2,4-Dinitranilin werden in 2600 Teilen Toluol mit 312 Teilen Chlorsulfonylisocyanat 4 Stunden bei 80°C gerührt. Die entstandene Suspension des N-2,4-Dinitrophenyl-N'-chlorsulfonylharnstoffs wird ohne Zwischenisolierung bei 25°C mit 360 Teilen Wasser versetzt und bei 60°C innerhalb von 4 Stunden zum 2,4-Dinitrophenylharnstoff verseift. Nach Verdünnen mit Wasser wird heiß filtriert und das Produkt mit heißem Toluol und Wasser gewaschen. Nach dem Trocknen im Vakuum erhält man 347 Teile 2,4-Dinitrophenylharnstoff vom Schmelzpunkt 195—197°C.

### Beispiel 2

370 Teile 6-Chlor-2,4-dinitranilin werden in 2600 Teilen Toluol mit 312 Teilen Chlorsulfonylisocyanat 4 Stunden bei 80°C gerührt. Die entstandene Suspension des N-6-Chlor-2,4-dinitrophenyl-N'-chlorsulfonylharnstoffs wird ohne Zwischenisolierung bei 25°C mit 360 Teilen Wasser versetzt und bei 60°C innerhalb von 4 Stunden zum 6-Chlor-2,4-dinitrophenylharnstoff verseift. Nach dem Verdünnen des Ansatzes mit Wasser wird mit 33 %iger Natronlauge neutralisiert, heiß filtriert und das Produkt mit heißem Toluol und Wasser gründlich gewaschen. Nach dem Trocknen im Vakuum erhält man 383 Teile 6-Chlor-2,4-dinitrophenylharnstoff vom Schmelzpunkt 219—221°C.

### Beispiel 3

Man verfährt nach den Angaben von Beispiel 2, jedoch mit dem Unterschied, daß die Umsetzung in 3000 Teilen Toluol-Mutterlauge durchgeführt wird. Aufarbeitung und Isolierung des Endproduktes

erfolgen nach den Angaben von Beispiel 2. Nach dem Trocknen im Vakuum erhält man 426 Teile 6-Chlor-2,4-dinitrophenylharnstoff vom Schmelzpunkt 213—215°C.

Beispiel 4

Man verfährt nach den Angaben von Beispiel 2, jedoch mit dem Unterschied, daß man die Suspension des gebildeten N-6-Chlor-2,4-dinitrophenyl-N′-chlorsulfonylharnstoffs bei Raumtemperatur abfiltriert und mit Toluol wäscht. Der so zwischenisolierte Chlorsulfonylharnstoff wird anschließend bei 50°C in 7000 Teile Wasser unter Rühren eingetragen. Dann werden, ebenfalls bei 50°C, 600 Teile Natriumhydrogencarbonat langsam unter Rühren eingetragen. Dabei verseift der Chlorsulfonylharnstoff innerhalb 3 Stunden. Der 6-Chlor-2,4-dinitrophenylharnstoff wird abfiltriert und mit 2000 Teilen 50°C warmem Wasser gewaschen.

Beispiel 5

40 Teile 6-Methyl-2,4-dinitranilin werden in 500 Teilen Toluol mit 34 Teilen Chlorsulfonylisocyanat 4 Stunden bei 80°C gerührt. Die Suspension des Chlorsulfonylharnstoffs wird mit 40 Teilen Wasser bei 25°C versetzt und bei 60°C zum 6-Methyl-2,4-dinitrophenylharnstoff verseift.

Aufarbeitung und Isolierung des Produkts erfolgen entsprechend den Angaben von Beispiel 2. Nach dem Trocknen im Vakuum erhält man 34 Teile 6-Methyl-2,4-dinitrophenylharnstoff vom Schmelzpunkt 216—218°C.

Beispiel 6

Die Umsetzung entspricht Beispiel 5, jedoch mit dem Unterschied, daß an Stelle von 6-Methyl-2,4-dinitroanilin 5-Methyl-2,4-dinitroanilin eingesetzt wird. Man erhält 42 Teile 5-Methyl-2,4-dinitrophenylharnstoff vom Schmelzpunkt 186—188°C.

Beispiel 7

641 Teile 5-Methoxy-2,4-dinitranilin werden in 3500 Teilen Toluol mit 510 Teilen Chlorsulfonylisocyanat 4 Stunden bei 80°C gerührt. Der gebildete Chlorsulfonylharnstoff wird nach Zugabe von 450 Teilen Wasser bei 25°C verseift. Aufarbeitung und Isolierung des Endproduktes erfolgen nach den Angaben von Beispiel 2. Nach dem Trocknen im Vakuum erhält man 758 Teile 5-Methoxy-2,4-dinitrophenylharnstoff vom Schmelzpunkt 212—215°C.

Beispiel 8

234 Teile 2,4-Dinitro-naphthylamin werden in 3500 Teilen Toluol mit 213 Teilen Chlorsulfonylisocyanat 6 Stunden bei 80°C gerührt. Die Suspension des gebildeten Chlorsulfonylharnstoffs wird bei 25°C mit 250 Teilen Wasser versetzt und bei 60°C in 4 Stunden verseift. Aufarbeitung und Isolierung des Endproduktes erfolgen nach den Angaben von Beispiel 2. Man erhält 237 Teile 2,4-Dinitronaphthylharnstoff vom Schmelzpunkt 214—216°C.

Beispiel 9

275 Teile 2,6-Dinitranilin werden in 2750 Teilen Toluol mit 319 Teilen Chlorsulfonylisocyanat 8 Stunden bei 80°C gerührt. Die Suspension des Chlorsulfonylharnstoffs wird bei 25°C mit 375 Teilen Wasser versetzt und bei 60°C verseift. Aufarbeitung und Isolierung des Endproduktes erfolgen nach den Angaben von Beispiel 2. Nach dem Trocknen im Vakuum erhält man 305 Teile 2,6-Dinitrophenylharnstoff vom Schmelzpunkt 215—217°C.

Beispiel 10

28 Teile 2-Nitranilin werden in 550 Teilen Toluol mit 34 Teilen Chlorsulfonylisocyanat 4 Stunden bei 80°C gerührt. Die Suspension des Chlorsulfonylharnstoffs wird bei 25°C mit 40 Teilen Wasser versetzt und bei 60°C verseift. Aufarbeitung und Isolierung des Endproduktes erfolgen nach den Angaben von Beispiel 2. Nach dem Trocknen im Vakuum erhält man 25 Teile 2-Nitrophenylharnstoff vom Schmelzpunkt 176—178°C.

Beispiel 11

33 Teile 4-Cyan-2-nitranilin werden in 750 Teilen Toluol mit 34 Teilen Chlorsulfonylisocyanat 1 Stunde bei 80°C gerührt.

Die Suspension des Chlorsulfonylharnstoffs wird nach Abkühlen auf 60°C mit 50 Teilen Wasser versetzt und bei 60°C verseift. Aufarbeitung und Isolierung des Endproduktes erfolgen nach den Angaben von Beispiel 2. Nach dem Trocknen im Vakuum erhält man 36 Teile 4-Cyan-2-nitrophenylharnstoff vom Schmelzpunkt 205—207°C.

**Patentansprüche**

1. Verbindungen der allgemeinen Formel

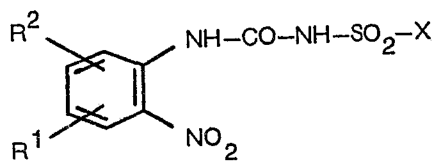

in der X Fluor oder Chlor, $R^1$ Wasserstoff oder Nitro und $R^2$ Wasserstoff, Fluor, Chlor, Brom, niederes Alkyl, niederes Alkoxy, Trifluormethyl, Cyan, Phenyl oder ein ankondensierter Benzolring ist.

2. Verbindungen nach Anspruch 1, in denen X Chlor und $R^2$ Wasserstoff, Chlor, Methyl, Methoxy, Cyan oder ein ankondensierter Benzolring ist.

3. Verfahren zur Herstellung der Verbindungen nach Anspruch 1, dadurch gekennzeichnet, daß man ein Amin der allgemeinen Formel

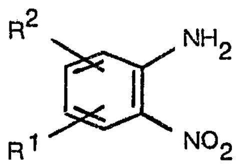

in der $R^1$ und $R^2$ die in Anspruch 1 angegebenen Bedeutungen haben, mit Fluor- oder Chlorsulfonylisocyanat umsetzt.

4. Verfahren nach Anspruch 3, dadurch gekennzeichnet, daß die Reaktion in Gegenwart eines inerten Löse- oder Verdünnungsmittels durchgeführt wird.

5. Verfahren nach Anspruch 3 und 4, dadurch gekennzeichnet, daß die Umsetzung bei einer Temperatur zwischen Raumtemperatur und dem Siedepunkt des inerten Löse- oder Verdünnungsmittels durchgeführt wird.

6. Verfahren nach Anspruch 3 bis 5, dadurch gekennzeichnet, daß die Umsetzung bei 40 bis 100°C durchgeführt wird.

7. Verfahren nach Anspruch 3 bis 6, dadurch gekennzeichnet, daß die Umsetzung mit Chlorsulfonylisocyanat durchgeführt wird.

8. Verfahren nach Anspruch 3 bis 7, dadurch gekennzeichnet, daß das Halogensulfonylisocyanat in geringem Überschuß eingesetzt wird.

9. Verwendung der Verbindungen nach Anspruch 1 zur Herstellung der entsprechenden Nitroarylharnstoffe durch Hydrolyse.

10. Verwendung der nach Anspruch 3 bis 9 erhaltenen Verbindungen zur Herstellung der entsprechenden Nitroarylharnstoffe durch Hydrolyse der Verfahrensprodukte in situ mit Wasser.

**Revendications**

1. Composés répondant à la formule générale:

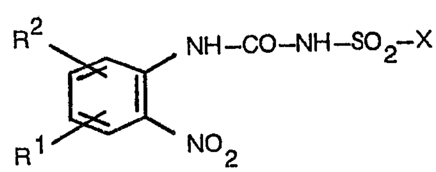

dans laquelle X représente le fluor ou le chlore, $R^1$ représente l'hydrogène ou le groupe nitro et $R^2$ représente l'hydrogène, le fluor, le chlore, le brome, un alkyle inférieur, un alcoxy inférieur, un radical trifluorométhyle, cyano ou phényle ou un noyau benzénique condensé.

2. Composés selon la revendication 1, dans lesquels X représente le chlore et $R^2$ représente l'hydrogène, le chlore, un radical méthyle, méthoxy ou cyano ou un noyau benzénique condensé.

3. Procédé de préparation des composés selon la revendication 1, procédé caractérisé en ce qu'on fait réagir une amine répondant à la formule générale:

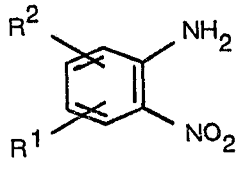

dans laquelle $R^1$ et $R^2$ ont les significations données à la revendication 1, avec l'isocyanate de fluorosulfonyle ou de chlorosulfonyle.

4. Procédé selon la revendication 3, caractérisé en ce qu'on effectue la réaction en présence d'un solvant ou diluant inerte.

5. Procédé selon l'une des revendications 3 et 4, caractérisé en ce qu'on effectue la réaction à une température comprise entre la température ambiante et le point d'ébullition du solvant ou diluant inerte.

6. Procédé selon l'une quelconque des revendications 3 à 5, caractérisé en ce qu'on effectue la réaction à une température comprise entre 40 et 100°C.

7. Procédé selon l'une quelconque des revendications 3 à 6, caractérisé en ce qu'on effectue la réaction avec l'isocyanate de chlorosulfonyle.

8. Procédé selon l'une quelconque des revendications 3 à 7, caractérisé en ce que l'isocyanate d'halogénosulfonyle est mis en jeu en un léger excès.

9. Application des composés selon la revendication 1 à la préparation des nitroaryl-urées correspondantes, par hydrolyse.

10. Application des composés obtenus selon l'une quelconque des revendications 3 à 9 à la fabrication des nitroaryl-urées correspondantes, par hydrolyse in situ, au moyen d'eau, des produits fournis par le procédé.

## Claims

1. Compounds of the general formula

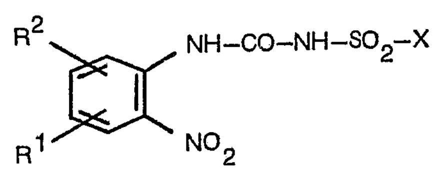

wherein X is fluorine or chlorine, $R^1$ is hydrogen or nitro and $R^2$ is hydrogen, fluorine, chlorine, bromine, lower alkyl, lower alkoxy, trifluoromethyl, cyano, phenyl or a fused benzene ring.

2. Compounds as claimed in claim 1, wherein X is chlorine and $R^2$ is hydrogen, chlorine, methyl, methoxy, cyano or a fused benzene ring.

3. Process for preparing the compounds as claimed in claim 1 which comprises reacting an amine of the general formula

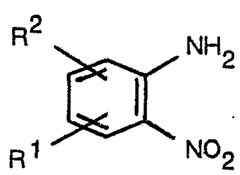

wherein $R^1$ and $R^2$ are as defined in claim 1, with fluoro- or chloro-sulfonyl isocyanate.

4. Process as claimed in claim 3, wherein the reaction is performed in an inert organic solvent or diluent.

5. Process as claimed in claim 3 or 4, wherein the reaction is performed at a temperature between room temperature and the boiling point of the solvent or diluent.

6. A process as claimed in claim 3 to 5, wherein the reaction is performed at 40 to 100°C.

7. Process as claimed in claim 3 to 6, wherein the reaction is performed with chlorosulfonyl isocyanate.

8. Process as claimed in claim 3 to 7, wherein a slight excess of the halogen sulfonyl isocyanate is reacted.

9. Use of the compounds according to claim 1 for preparing the corresponding nitroaryl ureas by hydrolysis.

10. Use of the compounds obtained according to claim 3 to 8 for preparing the corresponding nitroaryl ureas by hydrolyzing the said obtained compounds in situ with water.